# NEUE EUROPÄISCHE PATENTSCHRIFT

(11) **EP 1 343 864 B3**
(45) Veröffentlichungstag dieser Patentschrift: **03.11.2010**
(45) Hinweis auf die Patenterteilung: 10.11.2004
(21) Anmeldenummer: 01953658.0
(22) Anmeldetag: 26.07.2001
(51) Int. Cl.: C11D 3/39

(54) **REINIGUNGS- UND DESINFEKTIONSMITTEL**
CLEANING AND DISINFECTING AGENT
NETTOYANT ET DESINFECTANT

(30) Priorität: 13.10.2000 AT 17572000
(43) Veröffentlichungstag der Anmeldung: 17.09.2003
(73) Patentinhaber: Dipl.Ing. Thonhauser GmbH, 2380 Perchtoldsdorf (AT)
(72) Erfinder: THONHAUSER, Manfred, A-2380 Perchtoldsdorf (AT)
(74) Vertreter: KLIMENT & HENHAPEL
(86) Internationale Anmeldenummer: PCT/AT2001/000258
(87) Internationale Veröffentlichungsnummer: WO 2002/031098

(56) Entgegenhaltungen:
- WO-A-98/42812
- US-A- 3 677 953

## Beschreibung

Zur Reinigung und Desinfektion wird zur Zeit besonders Chlor verwendet. In wässriger Lösung bilden sich dabei Chlorverbindungen wie unterchlorige Säure (HOCl) oder Salzsäure (HCl), auf die letztendlich, gemeinsam mit dem dabei entstehenden Sauerstoff, die stark oxidierende, und daher desinfizierende Wirkung von wässrigen Chlorlösungen beruht. Gleichfalls desinfizierende Wirkung haben die bei der Reaktion von Chlor mit stickstoffhaltigen Verbindungen entstehenden Chloramine, die aber von manchen als geruchsintensiv und augenreizend empfunden werden. Kritische Nebenprodukte der Desinfektion mit Chlor sind schließlich Chlorkohlenwasserstoffe. Sie treten bei der Reaktion von Chlor mit organischem Material auf und können in höheren Konzentrationen gesundheitsschädlich werden. Es ist daher immer wieder versucht worden, Chlor durch andere Chemikallen für die Reinigung und Desinfektion zu ersetzen, ohne jedoch die Kelmtötungsgeschwindigkeit des Chlors zu erreichen.

Eine weitere Problematik beim Einsatz von Chlor zur Reinigung und Desinfektion stellt der Transport und die Lagerung dar, well bei dieser hochreaktiven Substanz spezielle Vorsicht geübt werden muss.

Ziel der Erfindung ist somit ein Reinigungs-und Desinfektionsmittel, das diese Nachteile bei gleichbleibender oxidierender und desinfizierender wirkung vermeidet.

Die Erfindung offenbart Reinigungs- und Desinfektionsmittel enthaltend wasserlösliches Permanganat, das zur Initierung der Oxidation organischer substanzen vorgesehen Ist, zusätzlich ein Mittel zur sicherstellung eines alkalischen Milieus mit einem pH-wert von mindestens 12, und weiter ein Oxidationsmittel, dessen Oxidationspotential über jenem von Mangan VII zu Mangan VI, vorzugsweise über jenem von HO₂⁻ zu OH⁻, liegt

Als weiteres Oxidationsmittel werden Peroxodisulfate verwendet.

Zum erreichen des alkalischen Milieus werden Alkalihydroxide verwendet.

Kaliumpermanganat (KMnO₄) ist ein starkes Oxidationsmittel, dessen keimtötende Wirkung seit langern bekannt ist. Im stark alkalischen Milieu beruht sie vor allem auf der Reduktion des siebenwertigen Mangans zur Oxidationsstufe +6. Aus unterschiedlichen Gründen ist eine Verwendung in Reinigungsund Desinfektionsmitteln aber nie gelungen. So erwies sich Kaliumpermanganat aufgrund seiner starken Oxidationswirkung beispielsweise unverträglich mit anderen notwendigen Inhaltsstoffen eines Reinigers. Außerdern wirkt Wasser beim hohen Oxidationspotential des Kaliumpermanganats als Reduktionsmittel, sodass sich Stabilitätsprobleme des Reinigers in wässriger Lösung ergeben.

GB 1 510 452 A offenbart ein Reinigungsmittel für Klosett-Schüsseln, das aus Kaliumpermanganat und einem Natriumalkylsulfat zur Verringerung der oberflächenspannung besteht. Keine weiteren Oxidationsmittel, insbesondere im Zusammenspiel mit Kaliumpermanganat, sind vorgesehen. Die Tauglichkeit des Mittels muss generell bezweifelt werden, weil keine Maßnahmen zur Sicherstellung des alkalischen Milieus ergriffen werden. Alkalische Bedingungen sind allerdings für das Vorbeugen einer Abscheidung des schwer wasserlöslichen Mangandioxids (Mn IV, "Braunstein") notwendig und begünstigen außerdem die keimtötende Wirksamkeit des Kaliumpermanganats.

WO-A-9 842 812 offenbart einen Reinigungsmitttelformkörper enthaltend Perkarbonat und Permanganat. Bei seiner Auflösung stellt sich ein pH-Wert oberhalb 10 ein.

Bei vorliegender Erfindung wird nun dem Permanganat ein Oxidationsmittel hinzugefügt, dessen Oxidationspotential jenes des Permanganats übersteigt. Vorzugsweise Wird dies durch Beigabe von Peroxodisulfaten, insbesondere Natriumperoxodisulfat, erreicht Wie noch genauer beschrieben wird, werden durch deren Zusammenwirken Radikalreaktionen initiert, in deren Folge es zur effizienten Oxidation organischer Substanzen kommt.

Durch die Verwendung von Alkalihydroxiden, vorzugsweise NaOH, wird eine Erhöhung der Keimtötungsgeschwindigkeit des Permanganats erreicht, weil die Oxidation organischer Verbindungen unter alkalischen Bedingungen beschleunigt wird.

Durch die Verwendung Oxidationsbeständiger Polyphosphate als Härtestabitisatoren, vorzugsweise Kalium-Tripolyphosphat, dann wird sicher geatellt, das die verwendeten Härtestabilisatoren (Komplexibildner) gegen Peroxodisulfate resistent sind. Außerdem ist eine gewisse Schutzwirkung gegen Korrosion von Buntmetallen und Kunststoffen anzunehmen.

Vorteilshafte Bedingungen für Transport und lagerung eines aus dem Verfahren resultierenden Reinigungs- und Desinfektionsmittel werden mit folgenden Zusammensetzung beschaffen:
20% - 35% 50% - Iges KOH, vorzugsweise 28%
5% - 25% 50% -Iges Kälium-Tripolyphosphat, vorzugsweise 15%
25 - 35.% Hypochloritlange, vorzugsweise 30%
mindestens 0,01 % KMnO₄

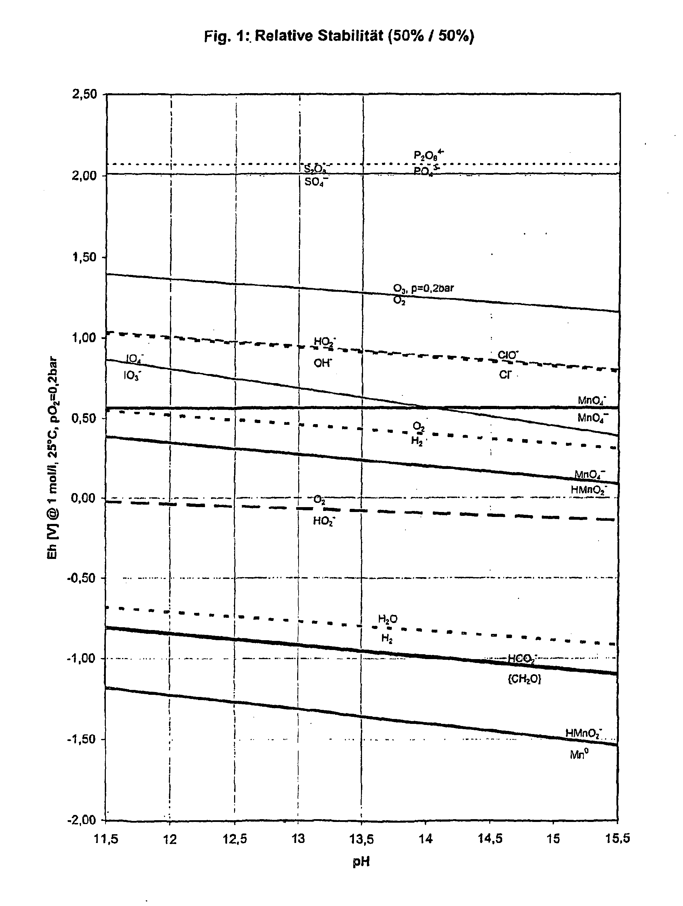

Die für die Wirksamkeit des erfindungsgemäßen Reinigungs-und Desinfektionsmittels relevanten Reaktionen werden nun unter Zuhilfenahme eines Pourbaix-Diagrammes (Fig. 1; für 25°C, 1 bar atmosphärischem Druck und einer Elektrolyt-Aktivität von 1 mol/l) im Detail beschrieben.

Zunächst wird ein starkes Oxidationsmittel in erfindungsgemäßer Form und Konzentration bereitgestellt, wobei es sich dabei vorzugsweise um ein Alkaliperoxodisulfat handelt. Obwohl das Alkaliperoxodisulfat ein starkes Oxidationsmittel ist, reagiert es bei Raumtemperatur und unter Abwesenheit entsprechender Katalysatoren mit organischen Verbindungen nur langsam. Die effiziente und vollständige Oxidation organischer Substanzen wird vielmehr durch das Kaliumpermanganat initiiert. Organischer Kohlenstoff wird dabei zu Oxalat oxidiert. Zur Beschleunigung der Reaktionskinetik zwischen Kaliumpermanganat und organischen Substanzen wird ein Alkalihydroxid beigegeben, vorzugsweise NaOH, um so ein alkalisches Milieu zu garantieren.

Bei der Anwendung der Erfindung wird das in Pulverform vorliegende Reinigungs- und Desinfektionsmittel zunächst in Wasser rasch und rückstandsfrei aufgelöst. Durch die erfindungsgemäße Zusammensetzung wird dabei darauf geachtet, dass die Auflösung des Härtestabilisators rasch genug erfolgt, um die Ausscheidung von Erdalkalikarbonaten und -hydroxiden aufgrund der ansteigenden Alkalität der Lösung zu verhindern, was besonders bei großen Wasserhärten entscheidend ist. Bei der Auflösung des erfindungsgemäßen Pulvers in Wasser erfolgt zunächst Oxidation von Hydroxid-Ionen, und zwar einerseits durch das Peroxodisulfat (GI. 1), andererseits aber auch durch das Permanganat (GI. 2), wobei siebenwertiges Mangan zu Mangan mit Oxidationszahl +6 reduziert wird. Dabei kommt es weiters zur Freisetzung von Sauerstoff.

Gl. 1: 3 OH⁻ + S₂O₈²⁻ = HO₂⁻ + 2 SO₄²⁻ + H₂O

Gl. 2: 4 OH⁻ + 4 MnO₄⁻ = O₂↑ + 4 MnO₄²⁻ + 2 H₂O

Das bei der Oxidation von Hydroxid-Ionen durch das Peroxodisulfat entstehende Wasserstoffperoxid-Ion kann allerdings eine Reoxidation des Mn(VI) zu Mn(VII) bewirken (Gl. 3):

Gl. 3: HO₂⁻ + 2 MnO₄²⁻ + H₂O = 3 OH⁻ + 2 MnO₄⁻

Wenn die Zersetzungsgeschwindigkeit des Peroxodisulfats mit jener des Permanganats nicht schritt halten kann, etwa weil die Zersetzung des Permanganats durch eine hohe Konzentration und/oder gute Oxidierbarkeit des organischen Stoffes begünstigt wird, wird es zu vermehrter Bildung von Mn(VI) kommen. Die Dominanz der sechswertigen Mangan-Spezies führt zu einer grünen Färbung der Lösung, im Gegensatz zu der anfänglichen violetten Färbung, die durch Mangan VII bewirkt wird. Die Oxidation organischer Verbindungen (hier mit "CH₂O" bezeichnet, was allgemein für Kohlenstoff der Oxidationsstufe 0 und im speziellen für ein Kohlehydrat steht) zu Oxalat durch Mn VII und die damit einhergehende Zersetzung des Permanganats erfolgt rasch, da der hohe pH-Wert auf zahlreiche organische Stoffe anionisierend wirkt, was den Angriff von anionischen Oxidationsmitteln erleichtert. Die Oxidation organischer Substanzen durch Mn VII involviert dabei auch MnO₄³⁻, wo Mangan mit der Oxidationszahl +5 vorliegt (Gl. 4), durch Permanganat aber wieder zu sechswertigem Mangan oxidiert wird (GI. 5).

Gl. 4: 2 {CH₂O} + 3 MnO₄⁻ + 2 H₂O = C₂O₄²⁻ + 3 MnO₄³ + 8 H⁺

Gl. 5: MnO₄³⁻ + MnO₄⁻ = 2 MnO₄²⁻

Der Angriff des Permanganats auf organische Substanzen gemäß GI. 4 bedingt allerdings nicht die hohe Wirksamkeit des erfindungsgemäßen Pulvers. Die schnelle und effiziente Oxidation organischer Stoffe wird vielmehr durch die nun startenden Radikalreaktionen verursacht. Ausgangspunkt ist dabei ein SO₄⁻-Radikal, das aus dem Peroxodisulfat hervorgeht. Dieses Radikal kann zunächst durch homolytische Spaltung des Peroxodisulfats (GI. 6), oder durch dessen Reaktion mit organischen Verbindungen entstehen (GI. 7):

Gl. 6: S₂O₈²⁻ = 2 **SO₄⁻**

Gl. 7: 2S₂O₈²⁻ + 2{CH₂O} + 2H₂O = 2SO₄²⁻ + 2**SO₄⁻** - +**{C⁺¹-R}** + 4H⁺

In Gleichung 7 bezeichnet dabei **{C⁺¹-R}** ein Radikal mit Kohlenstoff in der Oxidationsstufe +1, z.B. formal **{H₂C₂O₃}²⁻**, bei dem zwischen den Kohlenstoff-Atomen eine Doppelbindung besteht. Fettgedruckte Verbindungen kennzeichnen Radikale bzw. Radikalionen.

Wie Untersuchungsergebnisse zeigen, scheint allerdings das **SO₄⁻** in erster Linie durch das Zusammenwirken mit vorhandenen Mangan-Verbindungen zu entstehen. Es kann angenommen werden, dass Mangan VI bzw. Mangan V-Verbindungen radikalbildende Wirkung auf Peroxodisulfat gemäß der Reaktionen 8 und 9 haben:

Gl. 8: MnO₄²⁻ + C₂O₄²⁻ + 2H₂O = MnO₄³⁻ + 2CO₃²⁻ + 4H⁺

Gl. 9: MnO₄³⁻ + S₂O₈²⁻ = MnO₄²⁻ + SO₄²⁻ + **SO₄⁻**

Es wird nun eine Kaskade an Radikalreaktionen in Gang gesetzt, von denen im folgenden nur die wichtigsten genannt werden können. So bewirkt das **SO₄⁻**-Radikal etwa die Bildung von **OH-** Radikalen (G1. 10). Dieses Radikal zählt bekannterweise zu den reaktionsfähigsten Verbindungen und oxidiert organische Substanzen (GI. 11), wobei in weiterer Folge wieder **SO₄⁻-** Radikale entstehen können (GI. 12) :

Gl. 10: SO₄⁻ + H₂O = HSO₄⁻ + **OH.**

Gl. 11: 2 **OH**. + 2 {CH₂O} + H₂O = 2 OH⁻ + **{C⁺¹-R}** + 4 H⁺

Gl. 12: **{C+¹-R}** + 4S₂O₈²⁻ + H₂O = 4SO₄² + **4SO₄⁻** + C₂O₄²⁻ + 4H⁺

Das Hydroxid-Radikal kann nach seiner Bildung gemäß Gl. 10 allerdings auch mit Oxalat reagieren (GI. 13), wobei in weiterer Folge das Sulfatradikal durch Peroxodisulfat wieder hergestellt wird (GI. 14):

Gl. 13: **OH.** + C₂O₄²⁻ = OH⁻ + **C₂O₄⁻**

Gl. 14: **C₂O₄⁻** + S₂O₈²⁻ + 2 H₂O = 2 CO₃²⁻ + SO₄²⁻ + **SO₄⁻** + 4 H⁺

Ein anderer Reaktionskanal für die Oxidation organischer Verbindungen involviert das Sulfatradikal selbst. Das Sulfatradikal oxidiert organische Verbindungen (GI. 15) und kann schließlich durch Peroxodisulfat wieder nachgeliefert werden (GI. 16):

Gl. 15: 2 **SO₄⁻** + 2 {CH₂O} + H₂O = 2SO₄²⁻ + **{C⁺¹-R}** + 4H⁺

Gl. 16: **{C⁺¹-R}** + 4S₂O²⁻ + H₂O = 4SO₄²⁻ + 4**SO₄⁻** + C₂O₄²⁻ + 4H⁺

Auch das Sulfatradikal kann mit Oxalat reagieren (Gl. 17), wobei es wiederum mittels eines Peroxodisulfat-Moleküls nachgeliefert wird (GI. 18):

Gl. 17: **SO₄⁻** + C₂O₄²⁻ = SO₄²⁻ + **C₂O₄⁻**

Gl. 18: **C₂O₄⁻** + S₂O₈²⁻ + 2H₂O = 2CO₃²⁻ + SO₄²⁻ + **SO₄⁻** + 4H⁺

Es wird somit ersichtlich, dass es im Zuge des Ablaufens der Reaktionen 10-18 zu einer effizienten, da radikalvermittelten Oxidation organischer Verbindungen kommt, die durch Mangan-Verbindungen unterschiedlicher Oxidationsstufen eingeleitet und durch Peroxodisulfat aufrechterhalten wird.

Rekombinationsreaktionen zwischen Radikalen bringen die Kettenreaktionen 10-18 schließlich zum Abbruch (Gl. 19-24) :

Gl. 19: **SO₄⁻** + **SO₄⁻ =** S₂O₈2⁻

Gl. 20: **SO₄⁻** + **OH^{.}** = HSO₅⁻ (instabil)

Gl. 21: 4 **SO₄⁻** + **{C⁺¹-R}** + H₂O = 4 SO₄²⁻ + C₂O₄²⁻ + 4H⁺

Gl. 22: **OH^{.}** + **OH^{.}** = H₂O₂

Gl. 23: 4 **OH^{.}** + **{C⁺¹-R}** + H₂O = 4 OH⁻ + C₂O₄²⁻ + 4H⁺

Gl. 24: 3 **{C⁺¹-R}** + 3 H₂O = C₂O₄²⁻ + 4 {CH₂O} + 4 OH⁻

(Disproportionierung von z.B. **{H₂C₂O₃}²⁻**)

Da sich Manganat (VI) in Wasser thermodynamisch instabil verhält, kommt es in weiterer Folge zu einer Dominanz von Mangan II (Gl. 25):

Gl. 25: MnO₄²⁻ + H₂O = O₂↑ + HMnO₂⁻ + OH⁻

Eine Gelbfärbung der Lösung zeigt das Vorliegen von Mangan (II), das Oxalat-Komplexe bildet, an und somit auch den weitestgehenden Abschluss des Reinigungs- und Desinfektionsprozesses.

Während des gesamten Ablaufes der Kettenreaktionen 10-25 kommt es zur Freisetzung von Sauerstoff und Wasserstoffperoxid (Gl. 1, 2, 16 und 25), was den Reinigungs- und Desinfektionsprozess zusätzlich unterstützt.

Als zusätzliches, starkes Oxidationsmittel müssen nicht ausschließlich Peroxodisulfat-Verbindungen verwendet werden. Auch andere Oxidationsmittel, deren Oxidationspotential über jenem von Mangan VII zu Mangan VI (Linie MnO₄⁻/MnO₄⁻ im Pourbaix Diagramm von Fig. 1), vorzugsweise über jenem von HO₂⁻ zu OH⁻ (Linie HO₂⁻/ OH⁻ im Pourbaix Diagramm von Fig. 1), liegt, kommen dafür in Frage. Bezüglich der Linie MnO₄⁻/MnO₄⁻ wäre dazu etwa auch Periodat geeignet, das im Rahmen eines etwas modifizierten Chemismus für eine Reoxidation von Manganat V bzw. VI zu Permanganat sorgt. Die Verwendung von Peroxodlphosphat und Ozon ist zwar theoretisch denkbar, technisch jedoch kaum zu verwirklichen. Peroxodiphosphat ist derzeit in größeren Mengen nicht verfügbar und Ozon zersetzt sich aufgrund seiner hohen Reaktivität sehr rasch, wodurch es für kommerzielle Reinigungs- und Desinfektionsmittel nicht geeignet zu sein scheint. Hypochlorit wäre zwar in wässriger Lösung ausreichend stabil, die elektrochemische Dominanz des Redoxpaares ClO⁻/Cl⁻ ist zur Bildung von HO₂⁻ -Ionen jedoch auch bei längerer Lagerung sicherzustellen.

Alle Bestandteile des erfindungsgemäßen Reinigungs- und Desinfektionsmittels liegen dabei in Pulverform vor, was, abgesehen von der effizienten und raschen Oxidation organischer Substanzen, auch äußerst vorteilhaft für Lagerung und Transport des Mittels ist.

Die folgenden Beispiele sollen die Vielfältigkeit der Einsatzmöglichkeiten des erfindungsgemäßen Reinigungs- und Desinfektionsmittels dokumentieren und sind nicht im einschränkenden Sinn zu verstehen.

### Beispiel 1:

Das erfindungsgemäße Reinigungs- und Desinfektionsmittel kann besonders zielführend für Getränke-Schankanlagen verwendet werden. Das entsprechende Pulvergemisch enthält 58% NaOH (geprillt), 27.10% Kalium-Tripolyphosphat, 14.75% Natriumperoxodisulfat und 0.15% Kaliumpermanganat. Die Anwendung erfolgt in einer Konzentration von ungefähr 8 g Pulverprodukt pro Liter, wobei die Auflösung in Wasser rasch und rückstandsfrei erfolgt. Die Freisetzung von Sulfat-, Hydroxid- und anderen Radikalen sowie die hohe Alkalität begünstigen den Reinigungs- und Desinfektionsprozess. Der Farbumschlag von violett (Dominanz der Mangan (VII)-Spezies) auf grün (Dominanz der Mangan (VI)-Spezies) und schließlich auf gelb (Dominanz von Mangan (II/IV)) ermöglicht eine visuelle Beurteilung des Reinigungsfortschrittes.

### Beispiel 2:

Das erfindungsgemäße Reinigungs- und Desinfektionsmittel kann auch zur Flaschenreinigung eingesetzt werden. Derzeit werden verunreinigte Flaschen in Laugenbäder getaucht. Diese Bäder beinhalten im wesentlichen NaOH und Additive zur Verringerung der Oberflächenspannung und müssen auf mindestens 70°C erhitzt werden, um einen Reinigungsprozeß zu ermöglichen. Mit dem erfindungsgemäßen Reinigungs- und Desinfektionsmittel lässt sich die geforderte Keimtötung auch bei Raumtemperatur erzielen, was den maschinellen und finanziellen Aufwand reduziert. Die Flaschen werden lediglich mit einer in Wasser aufgelösten, erfindungsgemäßen Pulvermischung oder mit den zwei in flüssiger Form vorliegenden Komponenten NaOH/Kaliumtripolyphosphat und Peroxodisulfat/Permanganat besprüht. Nach einer Einwirkzeit, die sich aufgrund des Farbumschlages leicht optimieren lässt, werden die keimfreien Flaschen mit Wasser abgesprüht.

### Beispiel 3:

Anorganische Beläge in gemüse- und kartoffelverarbeitenden Betrieben oder Brauereien sind in der Regel sehr schwer löslich, da sie aus einem Gemisch von Salzen bestehen, die weder durch Mineralsäuren noch in Laugen gut löslich sind. Beispielsweise handelt es sich um Ca-Oxalate, Magnesiumammoniumphosphate oder Silicate. Das erfindungsgemäße Reinigungs- und Desinfektionsmittel ermöglicht hingegen die annähernd rückstandfreie Entfernung solcher Ausfällungen. Mit der erfindungsgemäßen Rezeptur wird eine wässrige Lösung von ca. 10% hergestellt und damit die zu reinigenden Oberflächen behandelt. Nach einer Einwirkzeit von weniger als 1 Stunde sind die Beläge leicht mit Wasser abspülbar.

## Patentansprüche

1. Reinigungs- und Desinfektionsmittel enthaltend wasserlösliches Permanganat, **dadurch gekennzeichnet, dass** das Mittel zusätzlich zum wasserlöslichen Permanganat, das zur Initiierung der Oxidation organischer Substanzen vorgesehen ist, zusätzlich ein Mittel zur Sicherstellung eines alkalischen Milieus mit einem pH-Wert von mindestens 12 enthält, und in Kombination mit mindestens einem weiteren Oxidationsmittel, dessen Oxidationspotential über jenem von Mangan VII zu Mangan VI, vorzugsweise über jenem von HO₂⁻ zu OH⁻, liegt, eingesetzt wird.

2. Reinigungs- und Desinfektionsmittel nach Anspruch 1, **dadurch gekennzeichnet, dass** als weiteres Oxidationsmittel Peroxodisulfate, vorzugsweise Natriumperoxodisulfat, verwendet werden.

3. Reinigung- und Desinfektionsmittel nach Anspruch 1 und 2, **dadurch gekennzeichnet, dass** als Permanganat Kaliumpermanganat verwendet wird.

4. Reinigungs- und Desinfektionsmittel nach Anspruch 1 bis 3, **dadurch gekennzeichnet, dass** zum Erreichen des alkalischen Milieus Alkalihydroxide, vorzugsweise NaOH, verwendet werden.

5. Reinigungs- und Desinfektionsmittel nach Anspruch 1 bis 4, **dadurch gekennzeichnet, dass** oxidationsbeständige Polyphosphate als Härtestabilisatoren, vorzugsweise Kalium-Tripolyphosphat, verwendet werden.

6. Reinigungs- und Desinfektionsmittel nach einem der Ansprüche 1 bis 5, **dadurch gekennzeichnet, dass** es in folgender Zusammensetzung verwendet wird:
50% - 70% NaOH, vorzugsweise 58%
20% - 35% Kalium-Tripolyphosphat, vorzugsweise 27%
10% - 20% Na₂S₂O₈, vorzugsweise 15%
mindestens 0,01% KMnO₄

7. Reinigungs- und Desinfektionsmittel nach einem der Ansprüche 1 bis 6, **dadurch gekennzeichnet, dass** sämtliche Bestandteile in Pulverform vorliegen.

8. Reinigungs- und Desinfektionsmittel nach einem der Ansprüche 1 bis 7, **dadurch gekennzeichnet, dass** pro Liter Reinigungs- und Desinfektionslösung 7-8 Gramm des Reinigungs- und Desinfektionsmittels aufgelöst sind.

9. Reinigungs- und Desinfektionsmittel nach einem der Ansprüche 1 bis 5, **dadurch gekennzeichnet, dass** es in folgender Zusammensetzung verwendet wird:
20% - 35% 50%-iges KOH, vorzugsweise 28%
5% - 25% 50%-iges Kalium-Tripolyphosphat, vorzugsweise 15%
25% - 35% Hypochloritlauge, vorzugsweise 30%
mindestens 0,01% KMnO₄

10. Reinigungs- und Desinfektionsmittel nach Anspruch 9, **dadurch gekennzeichnet, dass** es in 3% wässriger Lösung angewendet wird.

## Claims

1. A detergent and disinfectant containing water-soluble permanganate, **characterized in that** in addition to the water-soluble permanganate which is provided for initiating the oxidation of organic substances the agent additionally comprises an agent for securing an alkaline environment with a pH value of at least 12, and is used in combination with at least one further oxidant whose oxidation potential lies over that of manganese VII to manganese VI, preferably over that of HO₂⁻ to OH⁻.

2. A detergent or disinfectant as claimed in claim 1, **characterized in that** peroxodisulfates, preferably sodium peroxodisulfate, are used as a further oxidant.

3. A detergent or disinfectant as claimed in claim 1 and 2, **characterized in that** potassium permanganate is used as permanganate.

4. A detergent or disinfectant as claimed in claim 1 to 3, **characterized in that** alkali hydroxides, preferably NaOH, are used for achieving the alkaline environment.

5. A detergent or disinfectant as claimed in claim 1 to 4, **characterized in that** oxidation-resistant polyphosphates are used as hardness stabilizers, preferably potassium tripolyphosphate.

6. A detergent or disinfectant as claimed in one of the claims 1 to 5, **characterized in that** it is used in the following composition:
50% - 70% NaOH, preferably 58%
20% - 35% potassium tripolyphosphate, preferably 27%
10% - 20% Na₂S₂O₈, preferably 15%
at least 0.01% KMnO₄

7. A detergent or disinfectant as claimed in one of the claims 1 to 6, **characterized in that** all components are present in powder form.

8. A detergent or disinfectant as claimed in one of the claims 1 to 7, **characterized in that** 7 to 8 grams of the detergent and disinfectant are dissolved per liter of solution of detergent or disinfectant.

9. A detergent or disinfectant as claimed in one of the claims 1 to 5, **characterized in that** it is used in the following composition:
20% - 35% of 50% KOH, preferably 28%
5% - 25% of 50% potassium tripolyphosphate, preferably 15%
25% - 35% of hypochlorite lye, preferably 30%
at least 0.01% KMnO₄

10. A detergent or disinfectant as claimed in claim 9, **characterized in that** it is used in a 3% hydrous solution.

## Revendications

1. Produit détergent et désinfectant contenant du permanganate soluble dans l'eau, **caractérisé en ce que** le produit contient, en plus du permanganate soluble dans l'eau prévu pour initier l'oxydation de substances organiques, un produit garantissant un milieu alcalin d'un pH d'au moins 12, et qu'il est employé en combinaison avec au moins un autre agent oxydant dont le potentiel d'oxydation est supérieur à celui du manganèse passant de l'oxyde de manganèse (VII) à l'oxyde de manganèse (VI), de préférence supérieur à celui de HO₂⁻ passant à OH⁻.

2. Produit détergent et désinfectant selon la revendication 1, **caractérisé en ce que** l'on utilise comme agent oxydant supplémentaire du peroxodisulfate, de préférence du peroxodisulfate de sodium.

3. Produit détergent et désinfectant selon les revendications 1 et 2, **caractérisé en ce que** l'on utilise comme permanganate du permanganate de potassium.

4. Produit détergent et désinfectant selon les revendications 1 à 3, **caractérisé en ce que** l'on utilise des hydroxydes alcalins, de préférence NaOH, pour obtenir le milieu alcalin.

5. Produit détergent et désinfectant selon les revendications 1 à 4, **caractérisé en ce que** l'on utilise, pour stabiliser la dureté, des polyphosphates résistant à l'oxydation, de préférence du tripolyphosphate de potassium.

6. Produit détergent et désinfectant selon l'une quelconque des revendications 1 à 5, **caractérisé en ce que** sa composition est la suivante :
50 % - 70 % de NaOH, de préférence 58 %
20 % - 35 % de tripolyphosphate de potassium, de préférence 27 %
10 % - 20 % de Na₂S₂O₈, de préférence 15 %
au moins 0,01 % de KMnO₄

7. Produit détergent et désinfectant selon l'une quelconque des revendications 1 à 6, **caractérisé en ce que** tous les composants sont présents sous forme de poudre.

8. Produit détergent et désinfectant selon l'une quelconque des revendications 1 à 7, **caractérisé en ce que** sont dilués 7-8 grammes du produit détergent et désinfectant par litre de solution détergente et désinfectante.

9. Produit détergent et désinfectant selon l'une quelconque des revendications 1 à 5, **caractérisé en ce que** sa composition est la suivante :
20 % - 35 % de KOH à 50 %, de préférence 28 %
5 % - 25 % de tripolyphosphate de potassium à 50%, de préférence 15 %
25 % -35 % de solution d'hypochlorite, de préférence 30 %
au moins 0,01 % de KMnO₄

10. Produit détergent et désinfectant selon la revendication 9, **caractérisé en ce qu'**il est employé dans une solution aqueuse à 3%.
